# EUROPÄISCHE PATENTSCHRIFT

(11) **EP 1 753 343 B1**
(45) Veröffentlichungstag und Bekanntmachung des Hinweises auf die Patenterteilung: **19.12.2012**
(21) Anmeldenummer: 05735964.8
(22) Anmeldetag: 12.05.2005
(51) Int. Cl.: A61B 5/145, A61B 5/00

(54) **VERFAHREN ZUR MESSUNG DES TRANSKUTANEN CO2-PARTIALDRUCKS AN EINEM OHRLÄPPCHEN**
PROCESS FOR MEASURING PARTIAL TRANSCUTANEOUS CO2 PRESSURE AT AN EAR LOBE
PROCEDE POUR MESURER UNE PRESSION PARTIELLE DE CO2 TRANSCUTANEE SUR UN LOBE D'OREILLE

(30) Priorität: 18.05.2004 CH 867042004
(43) Veröffentlichungstag der Anmeldung: 21.02.2007
(73) Patentinhaber: Radiometer Basel AG, 4051 Basel (CH)
(72) Erfinder: GISIGER, Pierre-Alain, CH-2822 Courroux (CH); LIECHTY, Dominik, CH-4104 Oberwil (CH); EBERHARD, Patrick, CH-4103 Bottmingen (CH); KAGAWA, Sohei, Setagaya-ku 157-0065 (JP)
(74) Vertreter: Marnaes, Lene Meineche
(86) Internationale Anmeldenummer: PCT/CH2005/000265
(87) Internationale Veröffentlichungsnummer: WO 2005/110221

(56) Entgegenhaltungen:
- US-A- 4 539 994
- US-A- 4 805 122
- US-B1- 6 654 622
- LANG C J: "Apnea testing guided by continuous transcutaneous monitoring of partial pressure of carbon dioxide." CRITICAL CARE MEDICINE, Bd. 26, Nr. 5, Mai 1998 (1998-05), Seiten 868-872, XP009050520

## Beschreibung

Die vorliegende Erfindung bezieht sich auf ein Verfahren zur Messung des transkutanen CO₂-Partialdrucks an einem Ohrläppchen, wie es im Oberbegriff des unabhängigen Patentanspruchs 1 definiert ist, und auf einen Sensor zur Durchführung dieses Verfahrens, wie er im Oberbegriff des unabhängigen Patentanspruchs 9 definiert ist.

Um die respiratorischen Funktionen eines Patienten beurteilen zu können, ist häufig die Kenntnis seines arteriellen CO₂-Partialdrucks (paCO₂) erforderlich. Für die Messung des paCO₂-Werts stehen heute verschiedene Methoden zur Verfügung, wobei eine davon die Messung des transkutanen CO₂-Partialdrucks (tcpCO₂) beinhaltet. Diese indirekte Methode nutzt die Tatsache aus, dass CO₂ leicht durch Körpergewebe und Haut zu diffundieren vermag. Das Gas wird mit einem Sensor gemessen, der an der Hautoberfläche angebracht ist und der eine Heizung zum Erhitzen der auf der Haut aufliegenden Sensorauflagefläche aufweist. Wird die Sensorauflagefläche auf eine Temperatur von ca. 40°C bis 44°C erwärmt, so wird hierdurch eine lokale Erweiterung und Arterialisierung des Kapillarbetts an der Messstelle erzeugt. Unter dieser Bedingung weist der dort gemessene transkutane CO₂-Partialdruck eine hohe Korrelation zum arteriellen Wert auf. Dies ermöglicht mit gewissen Einschränkungen eine Bestimmung des paCO₂-Werts mit einer für die meisten Anwendungen ausreichenden Genauigkeit.

Die Bestimmung des paCO₂-Werts über die Messung des transkutanen CO₂-Partialdrucks bietet mehrere Vorzüge: Die Messung ist nicht invasiv, kontinuierlich und kann auch bei nicht intubierten Patienten eingesetzt werden.

Ein Verfahren und ein Sensor zur Messung des transkutanen CO₂-Partialdrucks an einem Ohrläppchen als vorteilhafter Messstelle sind in der US-B-6 654 622 beschrieben. Dabei wird der Sensor mittels einer Klammer oder eines Klebestreifens am Ohrläppchen befestigt. Dies erfordert keinen speziellen Aufwand und die Messstelle ist physiologisch zentral gelegen und beispielsweise für einen Anästhesisten während einer Operation zumeist leicht zugänglich und gut einsehbar. Ausserdem sind eine Behinderung des operierenden Chirurgen oder Probleme bezüglich der Sterilitätsanforderungen äusserst selten.

Bei Messungen des transkutanen CO₂-Partialdrucks an Ohrläppchen von Patienten mit in der US-B-6 654 622 beschriebenen Sensoren mit einer Temperatur der Sensorauflagefläche von ca. 41°C wurden nun jedoch während den ersten 20 Minuten oftmals unerwartet hohe Messwerte registriert. Die betreffenden Messkurven zeigen ein anfängliches Überschiessen der Messwerte, das ca. 5 Minuten nach Applikation des Sensors beginnt und nach weiteren ca. 5 Minuten wieder abzunehmen beginnt, bis es schliesslich ca. 20 Minuten nach Messbeginn nicht mehr zu erkennen ist. Direkte Vergleichsmessungen des arteriellen CO₂-Partialdrucks an entnommenen Blutproben haben ergeben, dass ca. 20 Minuten nach Messbeginn die erwarteten Messwerte des transkutanen CO₂-Partialdrucks erreicht werden.

Dieses Überschiessen des transkutanen CO₂-Partialdrucks in den ersten ca. 20 Minuten nach Messbeginn bedeutet, dass die Messwerte während dieser Zeit für die Bestimmung des arteriellen CO₂-Partialdrucks nicht ohne Weiteres übernommen werden können. Der arterielle CO₂-Partialdruck kann daher über dieses bekannte Verfahren zur Messung des transkutanen CO₂-Partialdrucks erst nach einer relativ langen Anlaufphase bestimmt werden.

Der vorliegenden Erfindung liegt daher die Aufgabe zugrunde, ein Verfahren zur Messung des transkutanen CO₂-Partialdrucks an einem Ohrläppchen zu schaffen, bei dem die gemessenen transkutanen CO₂-Partialdruck-Werte schon nach einer vergleichsweise kurzen Anlaufphase zuverlässig zur Bestimmung des arteriellen CO₂-Partialdrucks verwendbar sind. Ausserdem soll ein Sensor geschaffen werden, mit dem ein solches Verfahren durchführbar ist.

Diese Aufgabe wird durch das erfindungsgemässe Verfahren und den erfindungsgemässen Sensor gelöst, wie sie in den unabhängigen Patentansprüchen 1 und 9 definiert sind. Patentanspruch 1 bezieht sich auf ein erfindungsgemässes Verfahren zur Verhinderung eines Anstiegs des transkutanen CO₂-Partialdrucks in der Haut eines Ohrläppchens über den nach Stabilisierung erreichten CO₂-Partialdruck während der Arterialisierung der Haut. Bevorzugte Ausführungsvarianten ergeben sich aus den abhängigen Patentansprüchen.

Das Wesen der Erfindung besteht im Folgenden: Bei einem Verfahren zur Messung des transkutanen CO₂-Partialdrucks an einem Ohrläppchen erfolgt die Messung mittels eines Sensors, der eine Messeinrichtung zum Messen des transkutanen CO₂-Partialdrucks und eine Heizung zum Erhitzen einer zur Auflage am Ohrläppchen bestimmten Sensorauflagefläche aufweist. Bei dem Verfahren wird die Sensorauflagefläche erhitzt. Die Sensorauflagefläche wird während einer ersten Phase zur Verhinderung eines messungsbedingten Überschiessens des Messwerts des transkutanen CO₂-Partialdrucks über den nach Stabilisierung erreichten transkutanen CO₂-Partialdruck auf einer erhöhten Temperatur von mindestens 41,5°C gehalten. Danach wird die Temperatur der Sensorauflagefläche auf zwischen 37°C und 41°C gesenkt.

Dadurch, dass die Sensorauflagefläche während einer ersten Phase auf einer erhöhten Temperatur von mindestens 41,5°C gehalten wird, kann verhindert werden, dass der Messwert des transkutanen CO₂-Partialdrucks über den nach Stabilisierung erreichten transkutanen CO₂-Partialdruck überschiesst. Die Messwerte sind so wesentlich schneller verwertbar. Durch die nachfolgende Absenkung der Temperatur der Sensorauflagefläche auf zwischen 37°C und 41°C wird sichergestellt, dass der Patient an der Messstelle keine Verbrennungen erleidet.

Es wird vermutet, dass das anfängliche Überschiessen des Messwerts des transkutanen CO₂-Partialdrucks bei Messungen mit einer Sensorauflagefläche-Temperatur von zwischen etwa 37°C und 41°C darauf beruht, dass die an der Sensorauflagefläche anliegende Haut des Ohrläppchens zwar rasch erwärmt wird, was den lokalen Metabolismus erhöht und so zu einer erhöhten Freisetzung von CO₂ führt, aber die kutane Vasodilatation erst später erfolgt. Dadurch wird das metabolisch produzierte CO₂ erst verspätet über die Blutgefässe abgeführt und der transkutane CO₂-Partialdruck ist anfänglich überhöht. Ausserdem könnte das Überschiessen des Messwerts des transkutanen CO₂-Partialdrucks auch durch in der Haut gespeichertes CO₂ bewirkt oder verstärkt werden.

Mit Vorteil wird die Temperatur nach der Vorheizung mit der erhöhten Temperatur um mindestens 1°C, vorzugsweise mindestens 2°C, gesenkt. Dies ermöglicht die Wahl einer ausreichend hohen erhöhten Temperatur für die erste Phase, ohne dass es nach der Absenkung der Temperatur in der nachfolgenden zweiten Phase zu Hautverbrennungen kommt.

Bevorzugt liegt die erhöhte Temperatur im Bereich von 41,5°C bis 44°C. Dies reicht zur Verhinderung eines anfänglichen Überschiessen des Messwerts des transkutanen CO₂-Partialdrucks aus, ohne dass während der relativ kurzen ersten Phase die Gefahr von Hautverbrennungen besteht.

Mit Vorteil dauert die erste Phase mit der erhöhten Temperatur, während der die Sensorauflagefläche am Ohrläppchen anliegt, zwischen 5 und 60 Minuten. 5 Minuten erhöhter Temperatur reichen in den meisten Fällen aus, um ein Überschiessen des Messwerts des transkutanen CO₂-Partialdrucks zu verhindern. Wenn 60 Minuten erhöhter Temperatur nicht überschritten werden, können Hautverbrennungen praktisch ausgeschlossen werden.

Vorzugsweise dauert die erste Phase mit der erhöhten Temperatur zwischen 6 und 30 Minuten. Ein Überschiessen des Messwerts des transkutanen CO₂-Partialdrucks wird so wirksam verhindert. Mehr als 30 Minuten erhöhter Temperatur bringen diesbezüglich keinen Vorteil. Als optimal wird eine Dauer der ersten Phase von zwischen 6 und 15 Minuten angesehen. Eine unnötig lange erste Phase bringt keinen Vorteil.

Bei einer bevorzugten Ausführungsvariante dauert die erste

Phase zwischen 6 und 30 Minuten und die erhöhte Temperatur beträgt mindestens 42°C. Ein Überschiessen des Messwerts des transkutanen CO₂-Partialdrucks wird so wirksam verhindert und die erhöhte Temperatur wird nicht allzu lange aufrechterhalten.

Mit Vorteil beträgt die erhöhte Temperatur ca. 44°C und die erste Phase dauert vorzugsweise zwischen 6 und 20 Minuten. Unter diesen Bedingungen ist sichergestellt, dass der Messwert des transkutanen CO₂-Partialdrucks auf jeden Fall nicht überschiesst.

Vorteilhafterweise erfolgt die Senkung der Temperatur der Sensorauflagefläche mit einer Temperatursenkungsrate von kleiner oder gleich 1°C pro 10 s, vorzugsweise kleiner oder gleich 1°C/min. Wird die Temperatur zu schnell gesenkt, kann in der Messkurve ein kleiner Peak auftreten. D.h. die Messwerte des transkutanen CO₂-Partialdrucks werden durch die Temperatursenkung derart beeinflusst, dass deren Verwertung zur Bestimmung des arteriellen CO₂-Partialdrucks zu Ungenauigkeiten führt.

Ein weiterer Aspekt der Erfindung besteht in einem Verfahren zur Verhinderung eines Anstiegs des transkutanen CO₂-Partialdrucks in der Haut eines Ohrläppchens über den nach Stabilisierung erreichten CO₂-Partialdruck während der Arterialisierung der Haut bei der Messung des transkutanen CO₂-Partialdrucks. Bei diesem Verfahren wird die Haut an einer Messstelle des transkutanen CO₂-Partialdrucks während einer ersten Phase auf einer erhöhten Temperatur von mindestens 41,5°C gehalten und danach wird die Temperatur der Haut auf zwischen 37°C und 41°C gesenkt.

Wie bereits weiter oben erwähnt, wird bei der Messung des transkutanen CO₂-Partialdrucks an einem Ohrläppchen, bei dem die Sensorauflagefläche auf eine Temperatur von zwischen etwa 37°C und 41°C erhitzt wird, die an der Sensorauflagefläche anliegende Haut des Ohrläppchens rascher erwärmt, als eine kutane Vasodilatation, d.h. eine Arterialisierung der Haut, erfolgt. Dadurch wird das metabolisch produzierte CO₂ erst verspätet über die Blutgefässe abgeführt und der transkutane CO₂-Partialdruck ist anfänglich überhöht. Ausserdem ist in der Haut auch CO₂ gespeichert, das den transkutanen CO₂-Partialdruck anfänglich erhöht. Dem wirkt die erfindungsgemässe Lösung entgegen, dass die Haut an einer Messstelle des transkutanen CO₂-Partialdrucks während einer ersten Phase auf einer erhöhten Temperatur von mindestens 41,5°C gehalten und die Temperatur der Haut erst danach auf zwischen 37°C und 41°C gesenkt wird.

Ein erfindungsgemässer Sensor weist eine Messeinrichtung zum Messen des transkutanen CO₂-Partialdrucks und eine Heizung zum Erhitzen einer zur.Auflage an einem Ohrläppchen bestimmten Sensorauflagefläche auf. Er weist ausserdem eine Heizungssteuerung mit einem Zeitgeber auf, die die Heizleistung der Heizung nach einer vorbestimmten Zeitdauer reduziert, so dass die Temperatur der Sensorauflagefläche gesenkt wird. Mit einem solchen Sensor sind die erfindungsgemässen Verfahren durchführbar, was mit den erwähnten Vorteilen verbunden ist.

Mit Vorteil ist die vorbestimmte Zeitdauer, nach der die Heizungssteuerung die Heizleistung der Heizung reduziert, einstellbar. Dies ermöglicht es, die Dauer der ersten Phase erhöhter Temperatur einzustellen, beispielsweise im Hinblick auf den Patienten oder die Patientengruppe, bei dem bzw. der der Sensor angewandt wird. So ist es z.B. denkbar, bei Neugeborenen eine kürzere erste Phase zu wählen als bei Erwachsenen.

Die Messung des transkutanen CO₂-Partialdrucks selbst beruht im Allgemeinen auf einem elektrochemischen Prinzip. Sie erfolgt üblicherweise potentiometrisch, indem der pH-Wert einer dünnen Schicht einer Elektrolytlösung bestimmt wird, die über eine gut gasdurchlässige, hydrophobe Membrane mit der Haut in Verbindung steht. Eine Änderung des transkutanen CO₂-Partialdrucks an der Hautoberfläche bewirkt eine pH-Änderung der Elektrolytlösung, die sich proportional zum Logarithmus der tcpCO₂-Änderung verhält. Der pH-Wert wird beispielsweise durch Messung des Potentials zwischen einer Miniatur-pH-Glaselektrode und einer Ag/AgCl-Bezugselektrode bestimmt. Die vorliegende Erfindung ist aber nicht auf dieses Messverfahren beschränkt, sondern ist auch auf allfällige andere Verfahren zur Messung des transkutanen CO₂-Partialdrucks anwendbar.

Im Folgenden werden die erfindungsgemässen Verfahren und der erfindungsgemässe Sensor unter Bezugnahme auf die beigefügten Zeichnungen anhand von Ausführungsbeispielen detaillierter beschrieben. Es zeigen:
- Fig. 1 -: ein Diagramm mit Messkurven von Messungen des transkutanen CO₂-Partialdrucks an zwei Ohrläppchen einer Versuchsperson mittels Sensoren mit Sensorauflageflächen, wobei beim einen Ohrläppchen die Sensorauflagefläche des Sensors während einer ersten Phase auf einer erhöhten Temperatur von 42°C gehalten wird;
- Fig. 2 -: ein Diagramm mit Messkurven von weiteren Messungen des transkutanen CO₂-Partialdrucks an zwei Ohrläppchen derselben Versuchsperson, wobei beim einen Ohrläppchen die Sensorauflagefläche des Sensors während einer ersten Phase auf einer erhöhten Temperatur von 43°C gehalten wird;
- Fig. 3 -: ein Diagramm mit Messkurven von weiteren Messungen des transkutanen CO₂-Partialdrucks an zwei Ohrläppchen derselben Versuchsperson, wobei beim einen Ohrläppchen die Sensorauflagefläche des Sensors während einer ersten Phase auf einer erhöhten Temperatur von 44°C gehalten wird;
- Fig. 4 -: eine schematische Draufsicht auf einen erfindungsgemässen Sensor für die Messung des transkutanen CO₂-Partialdrucks und eine pulsoximetrische Messung der arteriellen Sauerstoffsättigung;
- Fig. 5 -: eine Schnittansicht des Sensors gemäss der Linie A-A in Figur 4;
- Fig. 6 -: eine Schnittansicht des Sensors gemäss der Linie B-B in Figur 4 und
- Fig. 7 -: eine Draufsicht auf die zur Auflage an einem Ohrläppchen bestimmte Sensorauflagefläche.

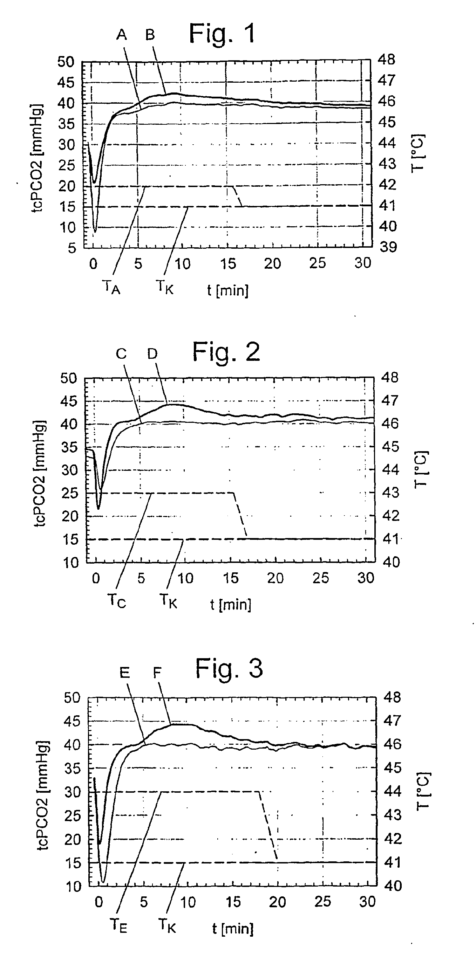

Bei den in den Fig. 1-3 dargestellten Diagrammen sind auf der x-Achse die Zeit in Minuten, auf der linken y-Achse der Partialdruck in mmHg und auf der rechten y-Achse die Temperatur in °C angegeben.

An einem Ohrläppchen einer Versuchsperson wurde der transkutane CO₂-Partialdruck jeweils mit einem Sensor gemessen, bei dem die Sensorauflagefläche des Sensors konstant auf einer Temperatur von 41°C gehalten wurde. Diese konstante Temperatur ist jeweils als gestrichelte horizontale Gerade T_{K} dargestellt. Die betreffenden Messwerte des transkutanen CO₂-Partialdrucks ergaben die Messkurve B in Fig. 1, die Messkurve D in Fig. 2 und die Messkurve F in Fig. 3. Bei allen diesen drei Messkurven B, D, F ist erkennbar, dass die Messwerte zunächst von ungefähr 28-35 mmHg auf etwa 18-23 mmHg stark abfallen und danach bis zu einem Peak von ungefähr 42-45 mmHg ansteigen, bevor sie wieder langsam auf einen ungefähr stabilen Wert von zwischen etwa 38 mmHg und 42 mmHg sinken.

Der erste starke Abfall der dargestellten Messwerte ergibt sich durch eine Entnahme des Sensors aus einer Eichkammer und dessen Ansetzen am Ohrläppchen. Dies bedeutet, dass die Messwerte der ersten 3-5 Minuten nicht physiologisch bedingt und daher für die Bestimmung des arteriellen CO₂-Partialdrucks unbrauchbar sind.

Danach kommt es zu einem Überschiessen der Messwerte des transkutanen CO₂-Partialdrucks über den am Schluss erreichten ungefähr stabilen Wert, das vermutlich, wie bereits weiter oben erläutert, darauf zurückzuführen ist, dass die an der Sensorauflagefläche anliegende Haut des Ohrläppchens zwar rasch erwärmt wird, was den lokalen Metabolismus erhöht und so zu einer erhöhten Freisetzung von CO₂ führt, aber eine kutane Vasodilatation erst später erfolgt. Dadurch wird das in der Haut metabolisch produzierte CO₂ erst verspätet über die Blutgefässe abgeführt und der transkutane CO₂-Partialdruck ist anfänglich überhöht. Ausserdem könnte das Überschiessen des Messwerts des transkutanen CO₂-Partialdrucks auch durch in der Haut gespeichertes CO₂ bewirkt oder verstärkt werden.

Um dieses Überschiessen zu verhindern, wurde erfindungsgemäss die Sensorauflagefläche des Sensors am anderen Ohrläppchen der Versuchsperson während einer ersten Phase auf einer erhöhten Temperatur von 42°C, 43°C bzw. 44°C gehalten und die Temperatur der Sensorauflagefläche erst nach Ablauf dieser ersten Phase auf 41°C gesenkt. Bei der in Fig. 1 dargestellten Messung dauerte die erste Phase ca. 16 Minuten und die Temperatur der Sensorauflagefläche (gestrichelte Temperaturkurve T_{A}) betrug während dieser Zeit 42°C. Danach wurde die Temperatur der Sensorauflagefläche innerhalb von einer Minute um 1°C gesenkt. Bei der in Fig. 2 dargestellten Messung dauerte die erste Phase ca. 15,5 Minuten und die Temperatur der Sensorauflagefläche (gestrichelte Temperaturkurve T_{C}) betrug während dieser Zeit 43°C. Danach wurde die Temperatur der Sensorauflagefläche innerhalb von eineinhalb Minuten um 2°C gesenkt. Bei der in Fig. 3 dargestellten Messung dauerte die erste Phase ca. 18 Minuten und die Temperatur der Sensorauflagefläche (gestrichelte Temperaturkurve T_{E}) betrug während dieser Zeit 44°C. Danach wurde die Temperatur der Sensorauflagefläche innerhalb von zwei Minuten um 3°C gesenkt.

Die den Temperaturkurven T_{A}, T_{C} und T_{E} zugeordneten Messwerte des transkutanen CO₂-Partialdrucks ergaben die Messkurve A in Fig. 1, die Messkurve C in Fig. 2 und die Messkurve E in Fig. 3. Bei allen diesen drei Messkurven A, C, E ist erkennbar, dass die Messwerte zunächst von ungefähr 30-35 mmHg auf etwa 8-28 mmHg stark abfallen und danach rasch bis auf einen ungefähr stabilen Wert von zwischen etwa 38 mmHg und 42 mmHg ansteigen. Ein Überschiessen der Messwerte des transkutanen CO₂-Partialdrucks über den am Schluss erreichten ungefähr stabilen Wert ist nicht zu erkennen. D.h. die Messwerte sind schon nach einer relativ kurzen Anlaufphase von ca. 5 Minuten verwertbar.

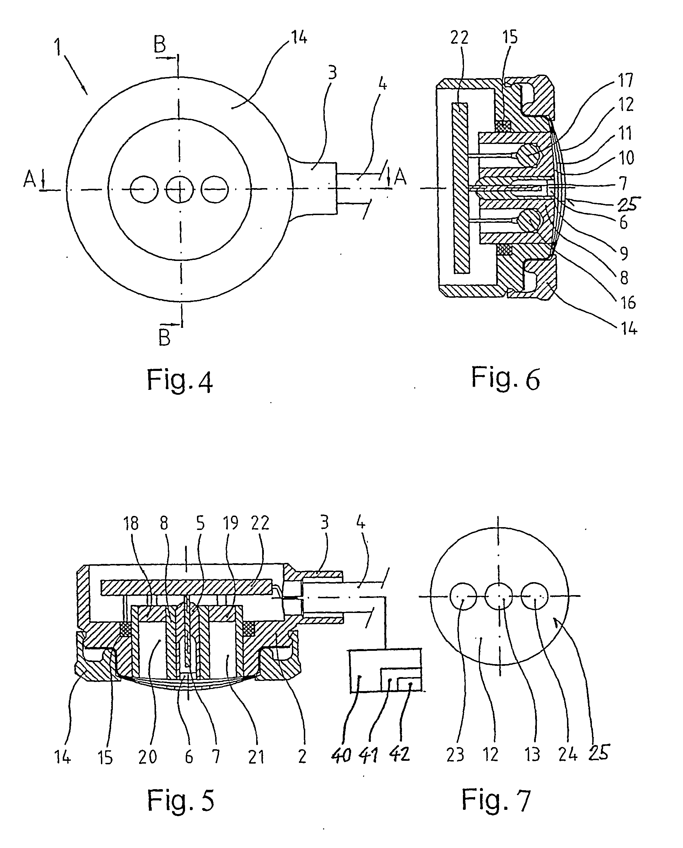

Die Fig. 4-7 zeigen ein Ausführungsbeispiel eines erfindungsgemässen Sensors für die Messung des transkutanen CO₂-Partialdrucks, das gleichzeitig auch eine pulsoximetrische Messung der arteriellen Sauerstoffsättigung durchführen kann. Fig. 4 zeigt eine Draufsicht auf einen Sensor 1, in der die Schnittflächen der Querschnittsansichten der Fig. 5 (A-A) und 6 (B-B) eingezeichnet sind. Der Sensorkopf besteht aus einem runden Gehäuse 2 aus Kunststoff mit einem halsförmigen Ansatz 3, durch den Verbindungsleitungen 4 zu einem in Fig. 5 schematisch dargestellten Messgerät 40 herausgeführt werden. In dem Gehäuse sind sowohl die für die Messfunktionen benötigten Komponenten als auch verschiedene elektronische Komponenten enthalten, wie im Folgenden beschrieben wird.

In der Mittelachse des Sensors befindet sich eine pH-Glaselektrode 5. Sie besteht aus einem zylinderförmigen Glasschaft, an dessen Stirnseite eine pH-empfindliche Glasschicht 6 eingeschmolzen ist. Im Inneren des Glaszylinders befindet sich eine innere Referenzelektrode mit einem im Glas eingeschmolzenen Zuleitungsdraht 7 aus Platin. Die pH-Elektrode 5 ist in einem Silberblock 8 eingebettet, dessen Oberfläche mit einer Chloridschicht 9 überdeckt ist. Die Oberfläche des Silberblocks bildet somit eine Ag/AgCl-Elektrode, die als Referenzelektrode für die pH-Wert Messung dient. Eine Elektrolytlösung, deren pH-Wert gemessen wird, befindet sich in einem porösen, hydrophilen Spacer 10, der mit einer gasdurchlässigen, hydrophoben Membrane 11 (beispielsweise Teflon) überzogen ist. Zum Schutz der Membrane gegen mechanische Verletzungen ist diese mit einer Metallblende 12 überdeckt. Diese Blende hat in der Mitte (über der pH-empfindlichen Glasschicht 6) eine Öffnung 13, durch die das zu messende CO₂-Gas in die Elektrolytlösung am Ort der pH-empfindlichen Glasschicht diffundieren kann. Der Spacer 10, die Membrane 11 und die Metallblende 12 sind mittels eines Spannringes 14 am Sensorgehäuse 2 befestigt. Der Silberblock 8 hat zusätzlich die Funktion eines Heizkörpers. Er ist mit einem Heizdraht 15 spulenförmig umwickelt, durch den er auf die für die transkutane Messung benötigte Temperatur von 37°C bis 45°C erwärmt wird. Wie in Fig. 6 zu erkennen ist, sind zwei Thermistoren 16 und 17 in zwei Bohrungen innerhalb des Silberblocks 8 eingebettet. Diese Thermistoren 16, 17 dienen zur Regelung und Kontrolle der gewählten Sensortemperatur. Aus der von den Thermistoren 16, 17 gemessenen Temperatur kann die Temperatur der Sensorauflagefläche berechnet werden, die im Normalfall geringer ist. Der bei der Berechnung anzuwendende Temperaturgradient kann vorgängig durch Messung der Temperatur an der Sensorauflagefläche bestimmt werden, wobei die Temperaturmessung durch den International Standard IEC 60601-2-23 Clause 42.3.104 normiert ist.

Die für die pulsoximetrische Messung benötigten optischen Komponenten sind im Querschnitt der Fig. 5 zu erkennen. Dies sind zwei LED 18, die dicht nebeneinander auf einem Keramikträger aufgebracht sind, und eine Fotodiode 19. Das Licht von und zu diesen Komponenten wird durch zwei zylinderförmige Lichtkanäle 20 und 21 geleitet, die aus zwei mit lichtdurchlässigem Material gefüllten Bohrungen im Silberblock bestehen.

Alle elektrischen Anschlüsse von und zu den verschiedenen Komponenten werden in eine elektronische Einheit 22 geführt, in der bereits ein Teil der Signalverarbeitung vorgenommen wird. Die Verbindungsleitungen 4 zum Messgerät 40 gehen von dieser elektronischen Einheit aus und werden, wie erwähnt, durch den Sensorhals 3 nach aussen geführt. Das Messgerät 40 weist eine Heizungssteuerungseinheit 41 mit einem Zeitgeber 42 auf, die die Heizleistung der durch den Silberblock 8 und den Heizdraht 15 gebildeten Heizung nach einer einstellbaren vorbestimmten Zeitdauer erfindungsgemäss reduzieren kann.

Figur 7 zeigt schliesslich eine Draufsicht auf die Metallblende 12, die die eigentliche Auflagefläche 25 des Sensors bildet. Wie bereits erwähnt, befindet sich in der Mitte der Blende eine Perforation 13, durch die das CO₂-Gas zum Elektrolyten an der pH-Elektrode gelangen kann. Die peripher dazu angeordneten zwei Perforationen 23 und 24 dienen dazu, das von den LED ausgehende und das vom Gewebe zurückgestreute Licht durchzulassen.

Zu den vorbeschriebenen erfindungsgemässen Verfahren und Sensoren sind weitere Variationen realisierbar. Hier ausdrücklich erwähnt sei noch, dass der Sensor selbstverständlich keine Komponenten zur Durchführung einer pulsoximetrischen Messung der arteriellen Sauerstoffsättigung aufweisen muss. Ausserdem kann auch die ganze Messelektronik im Sensorkopf selbst angeordnet sein.

## Patentansprüche

1. Verfahren zur Messung des transkutanen CO₂-Partialdrucks an einem Ohrläppchen mittels eines Sensors (1), der eine Messeinrichtung zum Messen des transkutanen CO₂-Partialdrucks und eine Heizung (8,15) zum Erhitzen einer zur Auflage am Ohrläppchen bestimmten Sensorauflagefläche (25) aufweist, bei welchem Verfahren die Sensorauflagefläche (25) erhitzt wird, wobei das Verfahren Folgendes umfasst:
- Halten der Sensorauflagefläche (25) während einer ersten Phase auf einer erhöhten Temperatur von mindestens 41,5 °C,
- und danach Senken der Temperatur der Sensorauflagefläche (25) um eine Temperatursenkungsrate bis zwischen 37 °C und 41 °C,
wobei
die Temperatursenkungsrate weniger als oder gleich 1°C pro 10₅ beträgt.

2. Verfahren nach Anspruch 1, wobei die Temperatursenkungsrate weniger als oder gleich 1,5 °C pro Minute beträgt.

3. Verfahren nach Anspruch 1, wobei die Temperatursenkungsrate weniger als oder gleich 1 °C pro Minute beträgt.

4. Verfahren nach einem der Ansprüche 1 bis 3, wobei die Temperatur um mindestens 1 °C, vorzugsweise um mindestens 2 °C gesenkt wird.

5. Verfahren nach einem der Ansprüche 1 bis 4, wobei die erhöhte Temperatur im Bereich von 41,5 °C bis 44 °C liegt.

6. Verfahren nach einem der Ansprüche 1 bis 5, wobei die erste Phase zwischen 6 und 30 Minuten dauert.

7. Verfahren nach Anspruch 6, wobei die erhöhte Temperatur mindestens 42 °C beträgt.

8. Verfahren nach Anspruch 7, wobei die erste Phase zwischen bis 6 und 20 Minuten dauert und die erhöhte Temperatur ca. 44 °C beträgt.

9. Sensor (1) zur Durchführung des Verfahrens nach einem der Ansprüche 1 bis 8, der eine Messeinrichtung zum Messen des transkutanen CO₂-Partialdrucks und eine Heizung (8,15) zum Erhitzen einer zur Auflage an einem Ohrläppchen bestimmten Sensorauflagefläche (25) aufweist, wobei der Sensor (1) eine Heizungssteuerung (41) mit einem Zeitgeber (42) aufweist, die geeignet ist, die Heizleistung der Heizung (8,15) nach einer vorbestimmten Zeitdauer so zu reduzieren, so dass die Temperatur der Sensorauflagefläche (25) um eine Temperatursenkungsrate bis zwischen 37 °C und 41 °C gesenkt wird, **gekennzeichnet dadurch, dass** die Heizungsstenerung (41) geeignet ist, die Heizleistung
so zu reduzieren dass die Temperatursenkungsrate weniger als oder gleich 1°C pro 10₅ beträgt.

10. Sensor nach Anspruch 9, wobei die vorbestimmte Zeitdauer einstellbar ist.

## Claims

1. A method for measurement of the transcutaneous CO₂ partial pressure on an earlobe by means of a sensor (1) which has a measuring device for measurement of the transcutaneous CO₂ partial pressure and a heating element (8, 15) for heating a sensor contact surface (25) intended for contact with the earlobe, during which method the sensor contact surface (25) is heated, wherein the method comprises the following:
- maintaining the sensor contact surface (25) at an elevated temperature of at least 41.5 °C during an initial phase
- and thereafter reducing the temperature of the sensor contact surface (25) at a temperature reduction rate to between 37 °C and 41 °C,
wherein
the temperature reduction rate is less than or equal to 1 °C per 10 seconds.

2. The method as claimed in claim 1, wherein the temperature reduction rate is less than or equal to 1.5 °C per minute.

3. The method as claimed in claim 1, wherein the temperature reduction rate is less than or equal to 1 °C per minute.

4. The method as claimed in one of the claims 1 to 3, wherein the temperature is reduced by at least 1 °C, preferably by at least 2 °C.

5. The method as claimed in one of the claims 1 to 4, wherein the elevated temperature is in the range of 41.5 °C to 44 °C.

6. The method as claimed in one of the claims 1 to 5, wherein the initial phase lasts between 6 and 30 minutes.

7. The method as claimed in claim 6, wherein the elevated temperature is at least 42 °C.

8. The method as claimed in claim 7, wherein the initial phase lasts between 6 and 20 minutes and the elevated temperature is approx. 44 °C.

9. A sensor (1) for implementation of the method as claimed in one of the claims 1 to 8 which has a measuring device for measurement of the transcutaneous CO₂ partial pressure and a heating element (8, 15) for heating of a sensor contact surface (25) intended for contact with an earlobe, wherein the sensor (1) has a heater control (41) with a timer (42), which is suitable for reducing the heating power of the heating element (8, 15) after a predefined period, such that the temperature of the sensor contact surface (25) is reduced by a temperature reduction rate to between 37 °C and 41 °C, wherein the heater control (41) is suitable for reducing the heating power such that the temperature reduction rate is less than or equal to 1 °C per 10 seconds.

10. The sensor as claimed in claim 9, wherein the predefined period is adjustable.

## Revendications

1. Procédé de mesure de la pression partielle transcutanée de CO₂ sur un lobe d'oreille au moyen d'un capteur (1) qui présente un dispositif de mesure pour mesurer la pression partielle transcutanée de CO₂ et un chauffage (8,15) pour chauffer une surface de contact de capteur (25) prévue pour le contact avec le lobe d'oreille, et procédé dans lequel la surface de contact de capteur (25) est chauffée, le procédé comprenant ce qui suit:
- le maintien de la surface de contact de capteur (25) à une température élevée d'au moins 41,5°C pendant une première phase
- puis l'abaissement de la température de la surface de contact de capteur (25) à une vitesse d'abaissement de la température jusqu'à atteindre une température comprise entre 37°C et 41 °C,
la vitesse d'abaissement de la température étant inférieure ou égale à 1 °C par 10s.

2. Procédé selon la revendication 1, **caractérisé en ce que** la vitesse d'abaissement de la température est inférieure ou égale à 1,5°C par minute.

3. Procédé selon la revendication 1, **caractérisé en ce que** la vitesse d'abaissement de la température est inférieure ou égale à 1 °C par minute.

4. Procédé selon l'une des revendications 1 à 3, dans lequel la température est abaissée d'au moins 1 °C, de préférence d'au moins 2°C.

5. Procédé selon l'une des revendications 1 à 4, dans lequel la température élevée se trouve dans la plage de 41,5°C à 44°C.

6. Procédé selon l'une des revendications 1 à 5, dans lequel la première phase dure entre 6 et 30 minutes.

7. Procédé selon la revendication 6, dans lequel la température élevée est d'au moins 42°C.

8. Procédé selon la revendication 7, dans lequel la première phase dure entre 6 et 20 minutes et la température élevée est d'environ 44°C.

9. Capteur (1) pour réaliser le procédé selon l'une des revendications 1 à 8, qui présente un dispositif de mesure pour mesurer la pression partielle transcutanée de CO₂ et un chauffage (8,15) pour chauffer une surface de contact de capteur (25) prévue pour le contact avec le lobe d'oreille, le capteur (1) présentant une commande de chauffage (41) avec un temporisateur (42) qui convient à la réduction de la puissance chauffante du chauffage (8, 15) après une durée prédéterminée de sorte que la température de la surface de contact de capteur (25) est abaissée à une vitesse d'abaissement de la température jusqu'à atteindre une température comprise entre 37°C et 41 °C, **caractérisé en ce que** la commande de température (41) convient à la réduction de la puissance chauffante de sorte que la vitesse d'abaissement de la température est inférieure ou égale à 1 °C par 10s.

10. Capteur selon la revendication 9, dans lequel la temporisation prédéterminée est réglable.
